# EUROPEAN PATENT SPECIFICATION

(11) **EP 1 762 629 B1**
(45) Date of publication and mention of the grant of the patent: **11.11.2009**
(21) Application number: 06018905.7
(22) Date of filing: 09.09.2006
(51) Int. Cl.: C12Q 1/68

(54) **Detection of biological DNA**
Nachweis biologischer DNA
Détection d'ADN biologique

(30) Priority: 12.09.2005 EP 05019758; 07.12.2005 EP 05026687
(43) Date of publication of application: 14.03.2007
(73) Proprietor: Roche Diagnostics GmbH, 68305 Mannheim (DE); F.HOFFMANN-LA ROCHE AG, 4070 Basel (CH)
(72) Inventor: Birkner, Christian, 82449 Uffing (DE); Koch, Thomas, 82377 Penzberg (DE); Moczko, Martin, 82377 Penzberg (DE); Von der Eltz, Herbert, 82362 Weilheim (DE)

(56) References cited:
- US-A1- 2004 176 584
- MAYR B M ET AL: "IDENTIFICATION OF BACTERIA BY POLYMERASE CHAIN REACTION FOLLOWED BY LIQUID CHROMATOGRAPHY-MASS SPECTROMETRY" ANALYTICAL CHEMISTRY, AMERICAN CHEMICAL SOCIETY. COLUMBUS, US, vol. 77, no. 14, 15 July 2005 (2005-07-15), pages 4563-4570, XP008059685 ISSN: 0003-2700

### SEQUENCE LISTING

<110> Roche Diagnostics GmbH
   F. Hoffmann-La Roche AG
<120> Detection of biological DNA
<130> 23307
<150> EP 05019758
   <151> 2005-09-12
<150> EP 05026687
   <151> 2005-12-07
<160> 14
<170> PatentIn version 3.2
<210> 1
   <211> 24
   <212> DNA
   <213> Artificial
<220>
   <223> primer
<400> 1
   gaaccttacc agatcttgac atcc 24
<210> 2
   <211> 18
   <212> DNA
   <213> Artificial
<220>
   <223> primer
<400> 2
   ccatgcacca cctgtcac 18
<210> 3
   <211> 83
   <212> DNA
   <213> Staphylococcus aureus
<400> 3
   gaaccttacc aggtcttgac atccttctga ccggcctaga gataggcttt ctcttcggag 60
   cagaagtgac aggtggtgca tgg 83
<210> 4
   <211> 85
   <212> DNA
   <213> Staphylococcus aureus
<400> 4
   gaaccttacc aaatcttgac atcctctgac ccctctagag atagagtttt ccccttcggg 60
   ggacagagtg acaggtggtg catgg 85
<210> 5
   <211> 85
   <212> DNA
   <213> Staphylococcus aureus
<400> 5
   gaaccttacc aaatcttgac atcctttgac aactctagag atagagcctt ccccttcggg 60
   ggacaaagtg acaggtggtg catgg 85
<210> 6
   <211> 85
   <212> DNA
   <213> Staphylococcus aureus
<400> 6
   gaaccttacc aaatcttgac atcctttgac aactctagag atagagcctt ccccttcggg 60
   ggacaaagtg acaggtggtg catgg 85
<210> 7
   <211> 85
   <212> DNA
   <213> Staphylococcus aureus
<400> 7
   gaaccttacc aaatcttgac atcctttgac aactctagag atagagcctt cctcttcggg 60
   ggacaaagtg acaggtggtg catgg 85
<210> 8
   <211> 85
<212> DNA
   <213> Staphylococcus aureus
<400> 8
   gaaccttacc aaatcttgac atcctttgac aactctagag atagagcttt ccccttcggg 60
   ggacaaagtg acaggtggtg catgg 85
<210> 9
   <211> 83
   <212> DNA
   <213> Staphylococcus aureus
<400> 9
   gaaccttacc aggtcttgac atccctctga cgactctaga gatagagttt tccttcggga 60
   cagaggtgac aggtggtgca tgg 83
<210> 10
   <211> 83
   <212> DNA
   <213> Staphylococcus aureus
<400> 10
   gaaccttacc aggtcttgac atcccgatgc tattcctaga gataggaagt ttcttcggaa 60
   catcggtgac aggtggtgca tgg 83
<210> 11
   <211> 83
   <212> DNA
   <213> Staphylococcus aureus
<400> 11
   gaaccttacc aggtcttgac atcctttgac cactctagag atagagcttt cccttcgggg 60
   acaaagtgac aggtggtgca tgg 83
<210> 12
   <211> 83
   <212> DNA
   <213> Staphylococcus aureus
<400> 12
   gaaccttacc aggtcttgac atcctttgac cactctagag atagagcttc cccttcgggg 60
   gcaaagtgac aggtggtgca tgg 83
<210> 13
   <211> 83
   <212> DNA
   <213> Staphylococcus aureus
<400> 13
   gaaccttacc aggtcttgac atcccgatgc ccgctctaga gatagagttt tacttcggta 60
   catcggtgac aggtggtgca tgg 83
<210> 14
   <211> 83
   <212> DNA
   <213> Staphylococcus aureus
<400> 14
   gaaccttacc aggtcttgac atcccgatgc ccgctctaga gatagagttt tacttcggta 60
   catcggtgac aggtggtgca tgg 83

## Description

### Field of invention

The present invention provides a method for the analysis of samples with respect to the presence of biological DNA. In particular, the present invention is directed to a method, that detects and identifies microorganisms potentially present in said sample.

### Prior art background

Biological and microbiological tests based on nucleic acid analysis are of still increasing importance for diagnostics, research and industrial applications. These kinds of tests are not only used to detect the presence of biological DNA in a sample, but also for its identification and quantification. Applications of said biological and microbiological tests can be found in disease recognition, environmental monitoring and quality control of reagents, therapeutics, cosmetics or food.

The nucleic acids are often present in very small concentrations and therefore, in the majority of cases, these tests comprise at least one amplification step of the characteristic DNA molecules. A well-known assay which entails the selective binding of two oligonucleotide primers is the polymerase chain reaction (PCR) described in US 4,683,195. This method allows the selective amplification of a specific nucleic acid region to detectable levels by a thermostable polymerase in the presence of deoxynucleotide triphosphates in several cycles.

Since the PCR results in a drastic amplification of nucleic acid molecules, even the slightest contamination of the sample with nucleic acid molecules from e.g. the reagents necessary for said amplification as well as a wrong design of primer molecules may result in a huge amount of undesired amplification products. A prominent example of such a phenomenon may occur in a microbiological test, where the existence of a bacterium in a sample should be tested and some of the used reagents are contaminated with this bacterium or with nucleic acids derived from this bacterium as well.

The design of primers is of utmost importance for the success of a certain biological or microbiological tests and mainly the specificity of the test can be adjusted by the primer design. On the one hand, primers may be used that amplify only a specific strain of a bacterium minimizing the risk of false positive results. On the other hand, primers may be used that amplify different types of bacteria and therefore, the risk of false negative results is minimized, but no information about the subspecies can be obtained.

The parallel detection, identification and quantification of biological DNA from different sources (e.g. from microorganisms, viruses) is usually performed using one or more primer pairs and different target specific fluorescent hybridization probes (e.g. hydrolysis or hybridization probes). The simultaneous identification of different DNA targets in these multiplex PCR assays can only be achieved by using multiple fluorescent dyes with different fluorescent properties. Because of the limited number of appropriate fluorescent dyes, in many cases an additional melting curve analysis of the probes must be performed for the identification of the different DNA targets.

The disadvantage of such multiplex PCR assays is the complexity of the reagent mixture, time consuming developments and high production costs. Moreover, the complexity of such detection mixtures often leads to a reduced sensitivity, because the probability of primer/probe interactions increases with the number of necessary primers and probes. A further limitation of probe based multiplex PCR assays is the inability to identify new and unknown species.

Another alternative to detect, identify and quantify a plurality of DNA molecules in parallel is the use of mass spectrometry (MS). The MS technique for the analysis of nucleic acids and especially of PCR products is well known in the art and the prior art relevant for the present invention is summarized in Mayr, B., et al., Anal. Chem. 77(14) (2005) 4563-4570. Using a mass spectrometer it is possible to identify DNA molecules by means of their molecular weight. If even the molecular weight is not sufficient to identify the DNA molecules, the tandem MS technique offers the additional opportunity to determine the sequence of the present molecules. However, the described MS based methods, especially for long PCR products, are expensive and the long analysis time is limiting its practical usability as a routine analysis tool.

### Brief description of the invention

Many alternatives for the analysis of samples with respect to the presence of biological DNA are known to someone skilled in the art, but none of these alternatives combine a real-time PCR with an optional analyzing step. Therefore, the present invention is directed to a method, a kit and a system for the analysis of biological DNA potentially present in said sample based on the combination of real-time PCR with an optional analyzing step.

One subject matter of the present invention is a method to analyze a sample with respect to the presence of biological DNA comprising the steps
a) performing a universal real-time PCR using Sybr Green, wherein potentially present biological DNA is amplified and
b) if biological DNA is amplified in step a), performing an additional analyzing step to analyze said amplified biological DNA, wherein said analyzing step is performed using a mass spectrometer (MS).

Within the scope of the present invention analyzing a sample comprises not only the detection but also the identification of biological DNA potentially present in said sample. Note that the analysis of a sample throughout the present invention is performed in two steps, first a pure detection step to verify if any biological DNA is present at all and second, an optional analyzing step to further analyze the detected biological DNA. To separate the universal real-time PCR detection step from the optional analyzing step has the advantage that said analyzing step is only performed if necessary, namely in case of any biological DNA amplified during the universal real-time PCR.

Throughout the present invention the phrase "universal real-time PCR" is used to emphasize that this PCR is not designed for the amplification of a single target molecule, but to amplify all target molecules within a certain group of targets.

The phrase "biological DNA" summarizes all kinds of DNA with biological origin. In particular, said biological DNA originates from microorganisms, such as bacteria, viruses or yeast.

The phrase "isolation step" summarizes all preparation steps that are necessary to extract the biological DNA potentially present in said sample in a form applicable for a subsequent PCR amplification. Therefore, said isolation step comprises e.g. a lysis step, if cell membranes have to be fractured prior to the real-time PCR amplification.

Throughout the present invention, the phrase "nucleic acid analyzer" summarizes all kinds of devices suitable to gain information about nucleic acid molecules, such as concentration, molecular weight, base composition or sequence.

Detection compounds for universal real-time PCR are all compounds that are able to bind unspecifically to nucleic acids and that are detectable in order to perform a nucleic acid quantification.

### Detailed description of the invention

One subject matter of the present invention is a method analyze a sample with respect to the presence of biological DNA comprising the steps
a) performing a universal real-time PCR using Sybr Green, wherein potentially present biological DNA is amplified and
b) if biological DNA is amplified in step a), performing an additional analyzing step to analyze said amplified biological DNA, wherein said analyzing step is performed using a mass spectrometer (MS).

Throughout the present invention all samples may be analyzed that have a chance to contain biological DNA. Without limiting the scope of the present invention, there are mainly three different applications, where the analysis of samples with respect to the presence of biological DNA is of interest.

The optional analyzing step is only performed if necessary, namely in case that biological DNA was amplified during the universal real-time PCR. Therefore, the method of the present invention is of advantage for screening applications, where only a minor fraction of the samples is expected to contain biological DNA. Such applications are quality controls, epidemic investigations as well as the detection of unanticipated or unknown species. For these applications the method of the present invention provides essential saving of costs, because the expensive analysis step is only performed, if biological DNA was detected during the real-time PCR. Moreover, depending on the user and/or the application the analysis step can be omitted even in case of a positive PCR result, if further information about the present DNA is not required. Last but not least, if the additional analysis step is performed only for a minor fraction of the samples, this has a positive effect with respect to throughput of a screening application.

Another subject matter of the present invention is a screening method for biological DNA comprising the steps
a) performing a universal real-time PCR using Sybr Green, wherein potentially present biological DNA is amplified and
b) if biological DNA is amplified in step a), performing an additional analyzing step to analyze said amplified biological DNA, preferably said analyzing step is performed using a mass spectrometer (MS).

In a preferred method according to the present invention, said sample is a biological sample, preferably blood, urine, tissue or food.

One application deals with the analysis of biological samples in order to detect and identify biological DNA as an indication of infections of e.g. humans or animals. In order to treat such infections effectively it is of course necessary to know the germ in detail and not just the information that some kind of infection is present. But on the other hand, if no infection is present at all, no further analyzing step is performed throughout the present invention. The method of the present invention is also applicable for food analysis, wherein the presence of e.g. microbial DNA is an indication that the food products are infected or spoilt. An important application of the present invention in the field of food analysis is the quality control of drinking water.

In another preferred method according to the present invention, said sample are reagents, preferably reagents of a diagnostic kit or buffer solutions.

In yet another preferred method according to the present invention, said sample are cosmetics, therapeutics or environmental samples.

Another application deals with biological DNA as contamination and therefore, the analysis according to the present invention is a test to verify the purity of said samples. In case of biological contaminations, it is not only of interest to know about the presence of said contamination, but especially the identification of said biological DNA is important in order to clarify its origin and to prevent such sample contamination in the future. Again, in the present invention no further analyzing steps is performed, if no contamination was detected at all.

An also preferred method according to the present invention comprises an additional isolation step that is performed prior to step a), wherein said isolation step isolates biological DNA from said sample.

In most cases it is necessary to separate said biological DNA from other components of the sample and therefore, it is preferred to perform one or more isolation steps that are known to someone skilled in the art prior to the PCR amplification. The most prominent example for this purpose is the use of DNA binding materials like glass surfaces due to their ability to reversibly bind DNA in the presence of chaotropic reagents (Vogelstein, B., and Gillespie, D., Proc. Natl. Acad. Sci. USA 76 (1979) 615-619).

In case of e.g. a microbial contamination of the sample, the microbial DNA can not be amplified directly. Therefore, in a preferred embodiment of the method according to the present invention said isolation step comprises an additional lysis step. Said lysis step fractures the cell membrane of e.g. bacteria and the subsequent isolation steps separate said microbial DNA from cell debris and buffer component prior to the real-time PCR amplification.

In a more preferred method according to the present invention, said isolation step isolates microbial DNA from potentially present microorganisms within said sample and said analyzing step identifies, which microorganism is present in said sample.

Here, the analysis of the amplified biological DNA is used to identify the microorganism present in said sample based on the known base composition or target sequence of its microbial DNA.

In another preferred embodiment of the method according to the present invention, said universal real-time PCR in step a) is performed with at least one primer amplifying biological DNA potentially present in said sample.

In yet another preferred embodiment of the method according to the present invention, said universal real-time PCR is performed with at least one primer pair.

In a more preferred embodiment of the method according to the present invention, said universal real-time PCR is performed with 2 - 5 primer pairs or with a generic primer pair.

The choice and the number of primers depend on the requested universality and uniformity of the amplification reaction. On the one hand, the universality of a primer mix can simply be improved by increasing the number of primers within the detection mix. But on the other hand, the probability of primer/probe interactions increases with the number of primers and probes and therefore, the sensitivity of the detection mix will be reduced.

An option to obtain universality without loosing the sensitivity of the detection mix is the use of generic primers. Generic primers are primers that are designed to amplify more than one biological target DNA in contrast to specific primers that are designed to amplify only one target DNA. In general, said generic primers are designed to detect all species of e.g. a certain bacteria in parallel in terms of amplifying a common part of their genome.

There are many different ways to realize real-time PCR. There are specific probes like labeled oligonucleotides that bind to other oligonucleotides having complementary sequences and unspecific probes like e.g. detectable double-stranded DNA binding moieties that intercalate into any double-stranded DNA. Throughout the present invention Sybr Green is used, because it detects the amplification of any biological DNA and therefore, reduces the risk of false-negative results.

In a method according to the present invention, said double-stranded DNA binding moiety is the fluorescence dye, SybrGreen.

Sybr Green is an the unspecific fluorescence dyes that intercalate into any double-stranded DNA. It changes its fluorescence properties upon intercalation and therefore, can indicate the amount of double-stranded DNA.

Using real-time PCR with a Sybr Green and e.g. a generic primer pair for analyzing samples with respect to biological DNA has the advantage that the risk of false-negative results is minimized. Throughout the present invention a real-time PCR with an Sybr Green and e.g. a generic primer pair is called universal real-time PCR, since this PCR is not designed for the amplification of a single target molecule, but to amplify all DNA molecules within a certain group of targets.

The use of such an universal real-time PCR has the disadvantage that no detailed knowledge about the amplified DNA can be gained. Therefore, an analytical technique providing good discriminative properties with respect to the oligonucleotide composition or sequences has to be used for the analyzing step, once the real-time PCR detection step indicated the general presence of biological DNA.

Another preferred method according to the present invention is a method, wherein said analyzing step comprises a melting curve analysis.

In many cases a melting curve analysis can be performed directly on the real-time PCR apparatus used for the amplification reaction. Here, the melting temperature of the double-stranded DNA is measured by continuously increasing the sample temperature while monitoring the fluorescence signals and the obtained melting temperature is an indication for the type of DNA that is present after amplification. Using the melting curve analysis it is e.g. possible to figure out, if the fluorescence intensity observed during the PCR amplification is based only on primer-dimer formation. Therefore, the melting curve analysis is an easy analyzing step to distinguish unwanted PCR products prior to the execution of any further analytical technique providing better discriminative properties.

Throughout the present invention, it a mass spectrometric technique is used for the analyzing step of the present invention. There are many different mass spectrometric techniques known to someone skilled in the art, but mostly two techniques are currently used for DNA analysis: Electrospray Ionization Mass Spectrometry (ESI-MS) and Matrix Assisted Laser Desorption Ionization Mass Spectrometry (MALDI-MS).

Yet another preferred method according to the present invention is a method, wherein said mass spectrometer (MS) is an electrospray ionization MS, a desorption electrospray ionization MS, a matrix-assisted laser desorption ionization MS, a fast atom bombardment MS, an ion-trap MS, triple quadrupol MS, time of flight (TOF) MS, quadrupol TOF MS, Fourier transform MS or combinations thereof.

Mass spectrometry generally involves the ionization of the analyte. The ionized target nucleic acid is usually generated by negatively charging the phosphate backbone via proton abstraction from the P-O-H groups. This involves running the mass spectrometer in negative mode. In ESI-MS, the analyte is initially dissolved in liquid aerosol droplets. Under the influence of high electromagnetic fields and elevated temperature and/or application of a drying gas the droplets get charged and the liquid matrix evaporates. After the evaporation of the entire liquid matrix, the charges remain localized at the analyte molecules that are transferred into the mass spectrometer. In MALDI-MS a mixture of analyte and matrix is irradiated by a laser beam. This results in localized ionization of the matrix material and the desorption of analyte and matrix. The ionization of the analyte is believed to happen by charge transfer from the matrix material in the gas phase.

Depending on the application it is possible to use any combination of the different MS techniques mentioned above in order to design the optimal experimental setup. Throughout the present invention it is especially preferred to use a combination of an ESI-MS with an ion-trap or a time of flight mass analyzer.

Also depending on the application it may be necessary to perform an additional separation step prior to the MS-analysis in order to separate the nucleic acids from other components of the sample. It is preferred to use a liquid chromatography (LC) for this purpose, such as a High Performance Liquid Chromatography (HPLC).

The MS-technique is very sensitive with respect to ingredients of the samples to be analyzed. Therefore, if the nucleic acid amplification product should optionally be analyzed by MS, one has to ensure that the amplification product does not contain ingredients that may interfere or even inhibit the MS-analysis.

In other words, changing parameters of a PCR amplification always requires an optimization with respect to the quality of the subsequent MS-analysis and the detection compounds for the real-time PCR of the present invention have to be chosen accordingly. It turned out that the unspecific fluorescence dye SybrGreen does not influence the subsequent MS-analysis and therefore, it is used throughout the present invention as dye for the real-time PCR.

A more preferred method according to the present invention is a method, wherein said mass spectrometer is used to obtain the molecular weight of said amplified biological DNA, preferably to obtain the sequence of said amplified biological DNA.

In many cases the detection of the weight of said amplified biological DNA is already sufficient to assign the origin of the DNA. But in some cases the molecular weight of said amplified biological DNA alone has not enough discriminative power to distinguish between e.g. two different species of a microorganism. Here, further knowledge about the DNA sequence is necessary.

In case of nucleic acids, the identification of the nucleic acid sequence can be obtained by the comparison of theoretical mass spectra from a given reference sequence with the experimental mass spectra measured by mass spectrometry. For this purpose, the reference sequence is systematically altered through permutation until a best fit between experimental and theoretical data is obtained. (WO 03/025219 A2, Oberacher, H., et al. Nucleic Acids Research 30(14) (2002) e67). Using this approach, it is possible to reliably verify sequences (re-sequencing) or to specifically detect oligonucleotides in a biological sample.

The MS/MS technique, also called tandem mass spectrometry, comprises the isolation of a parent molecular ion followed by fragment formation in the gas phase via collision or resonance activation and determining the molecular weights of the fragments. Application of tandem mass spectrometry for peptide sequence analysis is well known in the literature (US 6,017,693). The use of a MS/MS technique for DNA sequences is described in the article "Comparative sequencing of nucleic acids by liquid chromatography-tandem mass spectrometry" by Oberacher, H., et al. Analytical Chemistry 74 (1) (2002) 211-218.

An also more preferred method according to the present invention is a method, wherein said molecular weight of said amplified biological DNA or preferably said sequence of said amplified biological DNA is used to identify microorganisms present in said sample.

If the sequence of said amplified biological DNA is obtained by the mass spectrometric analysis step, it is possible to assign one or more microorganisms as the origin of said biological DNA using data base knowledge.

Yet another more preferred method according to the present invention is a method, wherein said at least one primer, said primer pairs or said generic primer pair amplifies fragments of said biological DNA with a length of below 200 base pairs, preferably below 130 base pairs.

Mass spectrometry is a powerful tool to analyze nucleic acids up to a certain amount of base pairs. Especially in case of sequencing with MS only rather short sequence lengths can be covered. Using e.g. the MALDI/Sanger method a maximum of 30 bp can be sequenced. This is also true for the method according to US-Patent 6,017,693, where problems are eminent already at NA lengths of 10 bp. The sequence verification according to WO 03/025219 A2 shows problems for oligonucleotides longer than 40 to 60 bp. Additionally, the comparison of theoretical data for all possible sequences with the experimental data for de-novo sequencing becomes time consuming with increasing nucleic acid length.

If longer biological DNA has to be analyzed, several different approaches are known in the art that offer the opportunity to produce nucleic acid fragments in a controlled fashion prior to the mass spectrometric analysis.

Controlled fragmentation of nucleic acids may be realized using base specific reagents, like e.g. digestion or restriction enzymes. In case of ribonucleic acids, several RNAses are known that are able to cleave the target molecules, e.g. the G-specific RNAse T₁ or A-specific RNAse U₁. Dicer enzymes (RNAseIII family) cut RNA into well defined pieces of about 20 bases. In case of DNA, it is possible to use e.g. the uracil-DNA-glycosylase (UDG) or restriction endonucleases that recognize a specific base sequence and cut within or nearby this region. Nick-endonucleases can be used to cut only one strand of a dsDNA double helix.

As an alternative, Gelfand et al (US 5,939,292) introduced a thermostable polymerase having reduced discrimination against ribonucleotides (NTPs or ribo-NTPs or ribo-bases). After an amplification step with said thermostable polymerase, the amplification product comprises a mixture of incorporated deoxyribonucleotides (dNTP) and NTPs providing the opportunity to use a simple alkaline hydrolysis step for the controlled fragmentation at the ribo-base positions. The resulting fragmentation products may be analyzed afterwards using electrophoresis in order to gain information of the nucleic acid sequence.

A fragmentation-based mass spectrometric method for the analysis of sequence variations is disclosed in WO 2004/050839. The WO 2004/097369 of the same applicant discloses a mass spectrometric method for the analysis and sequencing of biomolecules by fragmentation. The US 6,468,748 B1 of Genetrace Systems Inc. describes a method for the analysis of biomolecules comprising mass spectrometry and a fragmentation step. US 6,777,188 B1 discloses a method for genotyping a diploid organism comprising a comparison of masses and a cleaving step at modified nucleotides. Methexis Inc. describes a sequence analysis based on mass spectrometry, a cleavage reaction and the comparison with reference nucleic acids (WO 00/66771).

Still another preferred method according to the present invention is a method, wherein two or more of said universal real-time PCRs are performed in parallel and wherein each of said two or more universal real-time PCRs comprises different primers.

Since the amount of primers that can be used for a single PCR amplification is limited due to reasons outlined before, it is difficult to design a set of primers able to amplify e.g. gram(+) as well as gram(-) bacteria in a single PCR. Therefore, for the analysis of a sample potentially comprising a large amount of different contaminations, it is sometimes necessary to perform two or more universal real-time PCR according to the present invention. With respect to analysis throughput it is of course preferred to perform these two or more universal real-time PCR in parallel.

In a preferred method according to the present invention, each of said different primers is a mixture of 1 - 5 primer pairs or a generic primer pair.

As described before, there are several ways to perform each of said two or more universal real-time PCRs. It is necessary to provide at least one primer, preferably at least one primer pair amplifying all target molecules within a certain group of targets that are potentially present in said sample, wherein the choice and the number of primers depends on the requested universality and uniformity of the amplification reaction. Another preferred option is the use of generic primers to obtain universality without loosing the sensitivity of the detection mix.

In another method according to the present invention, at least 10, preferably more than 30, most preferably 96 or 384 universal real-time PCRs are performed in parallel.

Throughout the present invention it is preferred to use microtiter plates in order to perform said two or more universal real-time PCR in parallel. Only for those wells, where the universal real-time PCR indicates an amplification, a subsequent analysis step is performed.

A melting curve analysis may be performed directly in said microtiter plates. In case of a subsequent MS analysis, the amplification products must be transferred successively to the MS analyzer.

Usually, an eluent for the HPLC is required that is suitable for the separation of PCR amplicons from other components of the PCR reaction mixture (e.g. salts, enzymes, detergents). Possible eluents are combinations of organic solvents (e.g. acetonitrile) and water with appropriate amounts of ion pairing reagent (e.g. butyl-dimethylammonium bicarbonate). In addition, suitable standards and calibrators and an appropriate HPLC separation column may be used.

In case of e.g. bacterial detection, it may be necessary to use special grade reagents free of bacterial DNA in order to reach a high sensitivity. For the lysis of microorganisms like bacteria there are many different strategies known to someone skilled in the art depending on the sample material and it is only referred to the appropriate literature at this point.

In order to perform the method to analyze a sample with respect to the presence of biological DNA according to the present invention, several different reagents and a nucleic acid analyzer are necessary. For the purpose of real-time PCR, the reagents comprise at least a forward and a reverse primer, a thermostable DNA polymerase, nucleic acid triphosphates in buffer systems and detection compounds for the online detection of the amplification product. For a universal real-time PCR of the present invention, a SybrGreen is the detection compound. In case of starting from RNA molecules, the reagents additionally comprise a reverse transcriptase.

The reagents according to the present invention comprise reagents for an optional isolation step to gain nucleic acids without contaminations. Such reagents comprise e.g. a lysis buffer, a nucleic acid binding matrix, binding buffers, washing buffers and a nucleic acid elution buffer.

With respect to the reagents for an optional analyzing step, the necessary reagents are strongly depending on the used nucleic acid analyzer.

There are several nucleic acid analyzers known to someone skilled in the art that are suitable to determine the concentration, molecular weight, base composition or sequence of biological DNA as required by the present invention. Such nucleic acid analyzers comprise gel electrophoretic devices, melting curve analyzers, capillary sequencers and pyro sequencing devices.

In a more preferred embodiment according to the present invention, said nucleic acid analyzer is a mass spectrometer, preferably an electrospray ionization MS, a desorption electrospray ionization MS, a matrix-assisted laser desorption ionization MS, a fast atom bombardment MS, an ion-trap MS, triple quadrupol MS, time of flight (TOF) MS, quadrupol TOF MS, Fourier transform MS or combinations thereof.

Depending on the application it may be necessary to use combinations of the different MS techniques mentioned above in order to design the optimal experimental setup. In order to perform a mass spectrometric sequencing, it is advantageous to use a tandem mass spectrometer. Throughout the present invention it is especially preferred to use a combination of an ESI-MS with an ion-trap or a time of flight mass analyzer.

Also depending on the application, it may be necessary to use an additional separation device prior to the MS-analysis in order to separate the nucleic acids from other components of the sample. Throughout the present invention, it is preferred to use a liquid chromatography (LC) for this purpose, such as a High Performance Liquid Chromatography (HPLC).

Depending on the used mass spectrometer, the reagents according to the present invention may comprise HPLC eluents suitable for the separation of PCR amplicons from other components of the PCR reaction mixture (e.g. salts, enzymes, detergents). Possible eluents are combinations of organic solvents (e.g. acetonitrile) and water with appropriate amounts of ion pairing reagent (e.g. butyl-dimethylammonium bicarbonate).

If MALDI-MS is the nucleic acid analyzer, the reagents further comprise a matrix material.

### Description of the Figures

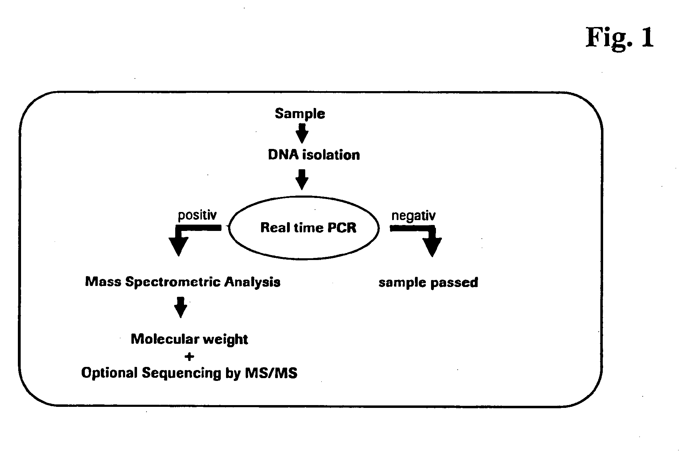

- **Figure 1:**: Flow chart of one embodiment of the present invention using mass spectrometry for the analysis step.
- **Figure 2:**: Genomic sequences of bacteria and their calculated molecular masses (Mᵣ), whereas the primer sequences are underlined.
- **Figure 3:**: PCR amplification curves of 4 different concentrations of bacterial DNA in 10 mM Tris-buffer, pH 8.0 (curve A: 100 geq; curve B: 10 geq; curve C: 1 geq; curve D: 0.5 geq) and a control amplification without bacterial DNA (curve E).
- **Figure 4:**: Melting curve analysis of the PCR products of Figure 3.
- **Figure 5:**: PCR amplification curves of 2 different concentrations of bacterial DNA in a sample comprising a fluorescent probe (curve A: 1 geq; curve B: 0.5 geq) and a control amplification without bacterial DNA (curve C).
- **Figure 6:**: Melting curve analysis of the PCR products of Figure 5.
- **Figure 7:**: Extracted Ion Chromatogram (TIC) of a PCR reaction mixture containing the amplicon of a *Staphylococcus aureus* contamination together with the corresponding mass spectrum of the amplicon (inset).
- **Figure 8:**: PCR amplification curves of a sample comprising a fluorescent probe and an unknown contamination (solid line A). As a control amplification, a known amount of bacterial DNA was added to said fluorescent probe (broken line B).
- **Figure 9:**: Melting curve analysis of the PCR products of Figure 8.
- **Figure 10:**: Extracted Ion Chromatogram (TIC) of a PCR reaction mixture containing the amplicon of an unknown bacterial reagent contamination.
- **Figure 11:**: Mass spectrum of the unknown bacterial amplicon.
- **Figure 12:**: Deconvoluted mass spectrum showing the MW of the unknown bacterial amplicon.

### Example 1

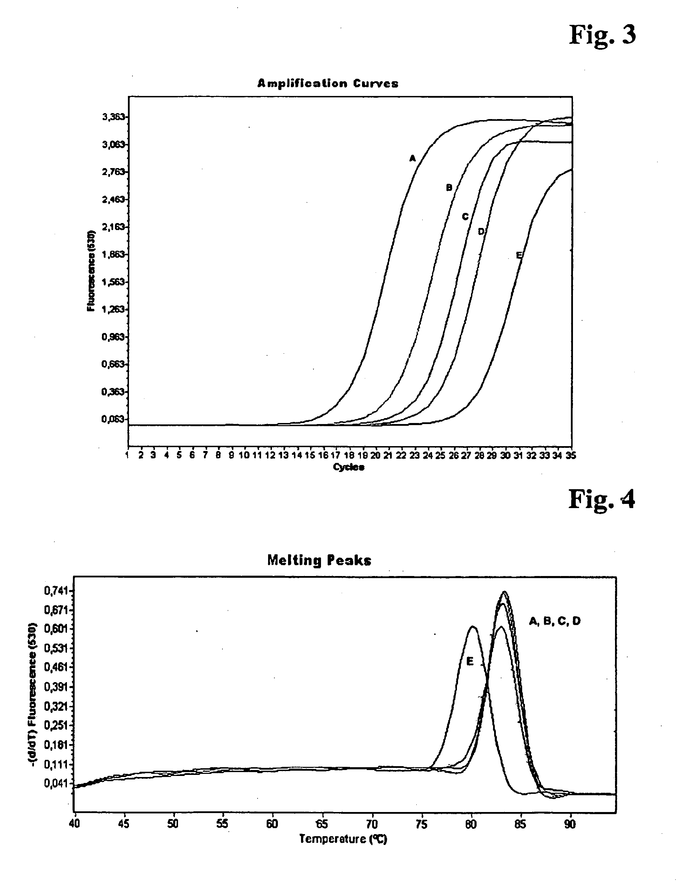

### Identification and quantification of a Staphylococcus aureus contamination in reagent mixtures (Figures 3-7):

The aim of the experiment was to test the ability of the present invention to detect a gram positive (gram(+) in the following) bacterial contamination in a reagent mixture containing a hybridization probe (named "Probe-Red 610" in the following, a 13-mer labeled with the fluorescence dye Red 610, c = 50 µM in 10 mM Tris pH = 8.0).

In order to simulate the possible degree of contamination, 1 and 0,5 geq of the S. aureus genomic DNA (in-house isolated from bacterium) were spiked to the reagent mixture.

As a standard, a dilution series of *S*. *aureus* genomic DNA was prepared with concentrations of c = 10² geq / 50 µl, c = 10 geq / 50 µl, c= 1 geq / 50 µl and c = 0.5 geq / 50 µl in elution buffer (10 mM TRIS ph 8.3). As a control, a PCR experiment without any target DNA (NTC = no template control) and of the pure reagent mix was performed.

Therefore, four types of amplification experiments were performed:
1) *S. aureus* genomic DNA spiked in a reagent mix at two different concentrations
2) *S*. *aureus* genomic DNA at different concentrations
3) NTC
4) Reagent mix

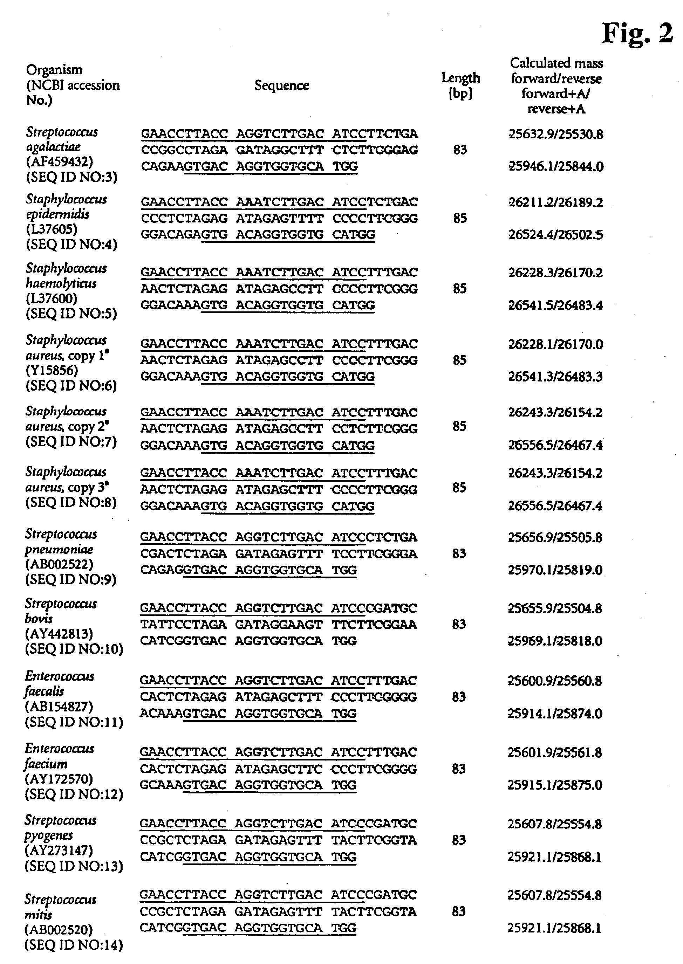

For the PCR experiments of this example, a generic primer mix was prepared to amplify a sequence from the 16S region of gram (+) bacteria, while meeting the requirement to amplify all gram (+) bacteria with equal sensitivity (see Figure 2 for a summary of the accordances of the primers with different G(+)-species).

Due to mismatches between primer and genomic sequences, however, different nucleotides were incorporated during amplification at the positions indicated by bold letters of Figure 2. The right column of the table in Figure 2 summarizes the calculated theoretical mass of the forward and reverse amplicons. In addition, the molecular weight of commonly observed 5'-no-template adenosine addition products is given in the right column of the table in Figure 2.

For some species identified by in Figure 2, multiple copies of the 16S rRNA gene exist, which do not have identical sequences at the positions indicated by bold letters. Note that the 16S rRNA copies 2 and 3 nevertheless have identical base compositions, and therefore they have the same molecular weight.

For said PCR experiments, the primers were used in a custom made DNA free master mix solution (LightCycler UniTool 100 v.3 Kit, Roche Diagnostics GmbH, Mannheim, Cat.No. 03 585 727) comprising a FastStart Enzyme (bottle 1a), a Reaction Mix (bottle 1b), a MgCl₂ solution (bottle 2: 25 mM, M-grade) and PCR-M-grade water (bottle 3). The amplification experiments were carried out with a LightCycler^{®} (LC) 2.0 instrument (Roche Diagnostics GmbH, Mannheim).

| **component** | **volume** |
|---|---|
| H₂O | 135.00 µl |
| MgCl₂ (25 mM) | 432.00 µl |
| I a | 51.00 µl |
| 1b | 669.00 µl |
| Primer fwd 5 µM | 252.00 µl |
| Primer rev 5 µM | 252.00 µl |
| SYBR Green (1:100 in water) | 9.00 µl |
| Master mix volume | 1800 µl |

50 µl of the master mix were pipetted in a 100 µl LC-capillary and 50 µl of the standard (*S. aureus* genomic DNA) were added.

The amplification experiments, divisible into denaturation, amplification and melting curve analysis, were performed as follows:
- The initial denaturation was performed at 95 °C for 10 minutes, whereas the heating was conducted with a rate of 20 °C/s.
- The amplification took place during 45 PCR cycles. Each cycle had the following temperature profile: 95 °C for 20 s (transition rate: 20 °C/s), 60 °C for 25 s (transition rate: 20 °C/s) and 72 °C for 40 s (transition rate: 20 °C/s). A single fluorescence measurement was performed at the end of each of the amplification cycles 11 to 45.
- Prior to the melting curve analysis the sample was cooled down to 40 °C (transition rate: 20 °C/s) for 60 s. Afterwards, the melting curve was recorded with a continuous fluorescence measurement during the heating of the sample to 95 °C (transition rate: 0.1 °C/s).

The detection was performed using SybrGreen (Roche Diagnostics GmbH, Mannheim, ID-Nr.: 11988131001 in a 1:100 dilution in PCR-grade water) and the reactions were monitored in real time using the SybrGreen modus of the LightCycler® instrument according to the manufacturer's instructions.

Following the PCR amplification, the content of the capillary reaction vessels containing a PCR product were opened and subjected to MS analysis.

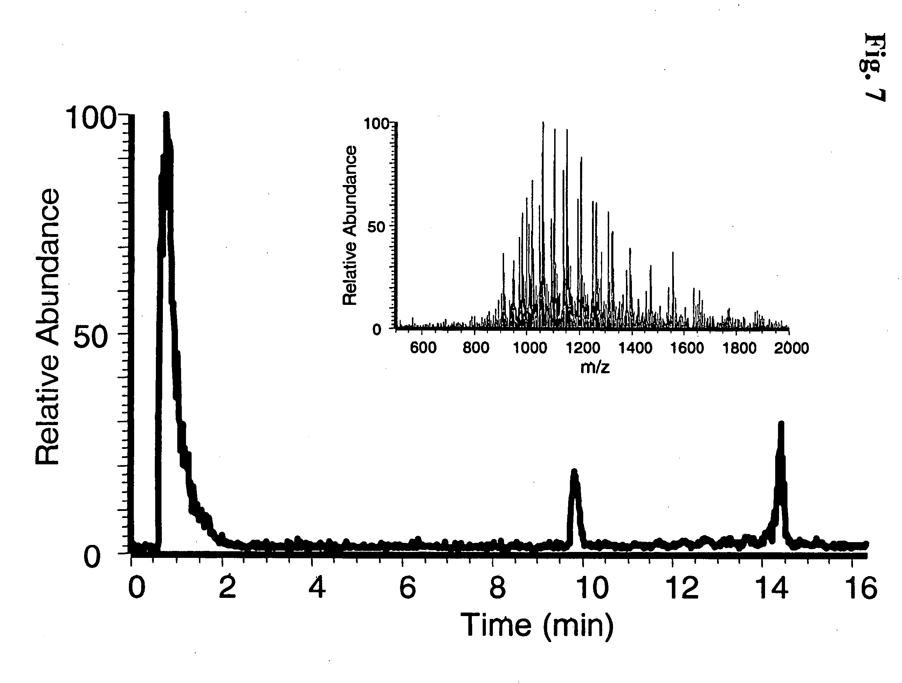

500 nL of the mixtures were separated by High Performance Liquid Chromatography (HPLC) and analyzed by Tandem-Mass Spectrometry. A commercially available computer controlled integrated HPLC system was used (model Surveyor, Thermo Electron Corp., San Jose, CA). The system consisted of a gradient micro pump an autosampler and a microinjector valve with a 500 nL internal sample loop. The 50 mm 0.2 mm i.d. monolithic capillary column was from LC-Packings (Sunnyvale, CA). A flow-rate of 2.0 µL/min through the column was split from a primary flow of 250 µL/min by means of a T-piece and a fused silica restriction capillary. A binary eluent system was used, with eluent A: 25 mM BDMAB in water (pH 9), and B: 25 mM BDMAB in water/acetonitrile (40:60) pH 9. The gradient for separation of the PCR reaction mixture was 100% A (0-5 min), 100 →20 % A (5-15 min) and 20 % A (15-20 min). The HPLC column was heated to 65 °C for strand denaturation and directly connected to the electrospray capillary (fused silica, 90 µm o.d., 20 µm i.d., Polymicro Technologies, Phoenix, AZ). ESI-MS was performed on an ion trap mass spectrometer (LCQ Deca XP, Thermo Electron Corp., San Jose, CA) equipped with an electrospray ion source. An electrospray voltage of -3.0 kV was employed and the heated capillary temperature was set to 200 °C. Total ion chromatograms and mass spectra were recorded on a personal computer with the data analysis software Xcalibur ver. 1.4 (Thermo Electron). Mass calibration and coarse tuning were performed using a mixture of Ultramark, caffeine and MRFA (Thermo Electron Corp., San Jose, CA). Fine tuning for ESI-MS of oligodeoxynucleotides in the negative ion mode was performed with a 24-mer oligodesoxythymidin (dT₂₄). Using this experimental set-up the molecular weight (MW) of amplification products was determined. The comparison with the calculated molecular weight of the corresponding *S*. *aureus* amplicon allowed for clear identification of contaminated samples. The NTCs did not exhibit signals in the expected range and spiked samples were positive for *S. aureus down to ca. 0.5 geq*/PCR (see Figure 7).

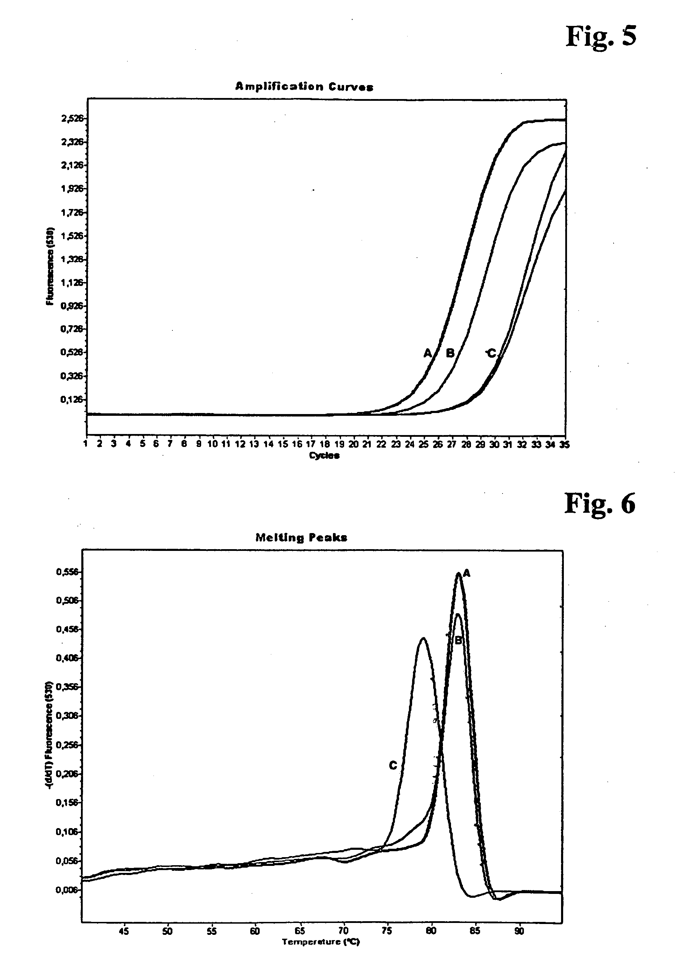

The Cₚ-values of the quantification curves of the amplification experiment in figures 3 and 4 show the linearity and sensitivity of this experiment. The positive signal of the no template control (NTC) is a result of a primer/dimer formation and can be distinguished from a G(+) bacterial contamination via melting curve analysis (figures 4 and 6) or via LC-MS/MS.

### Example 2

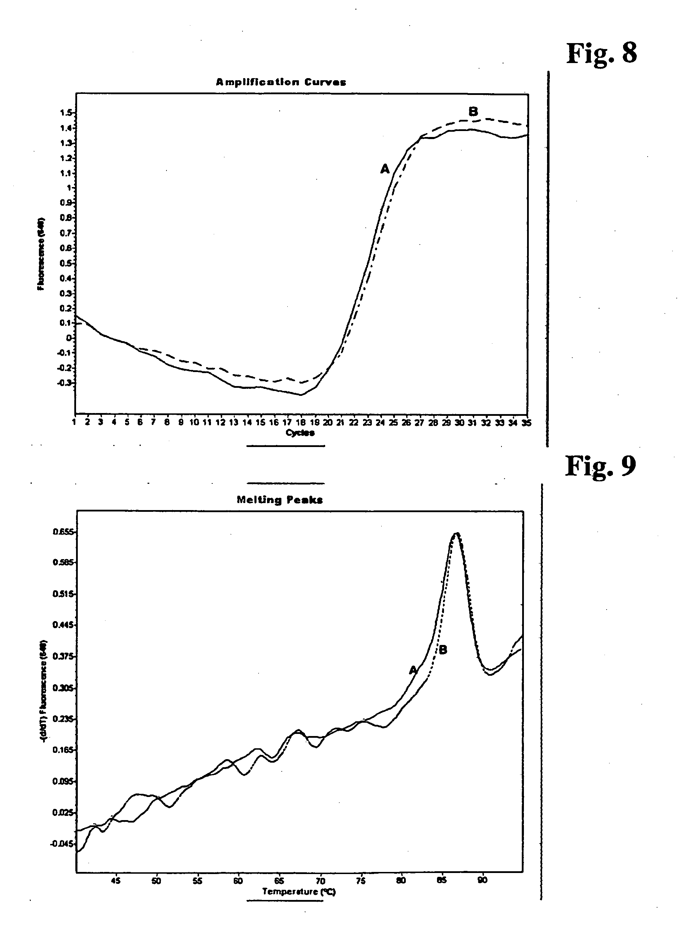

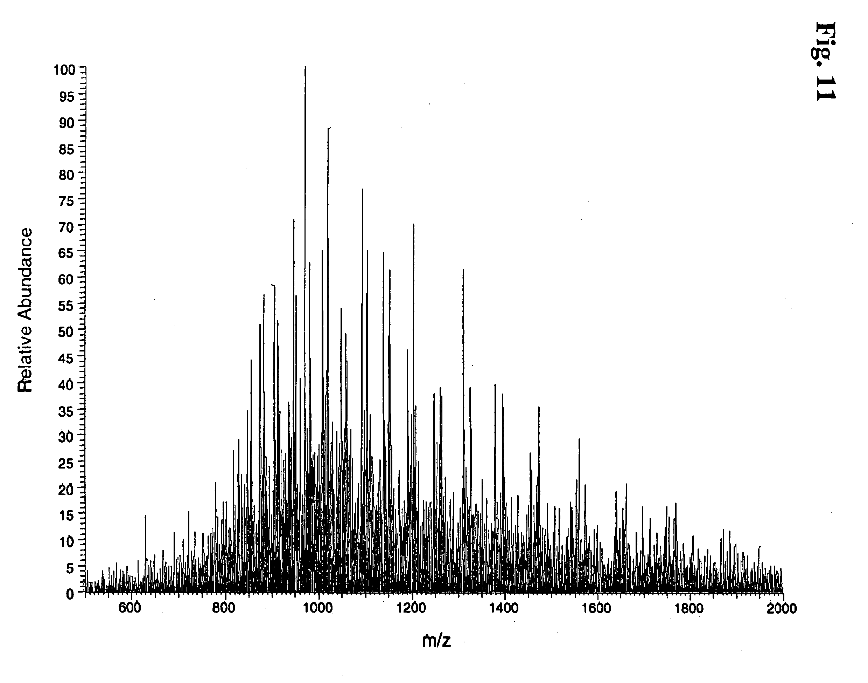

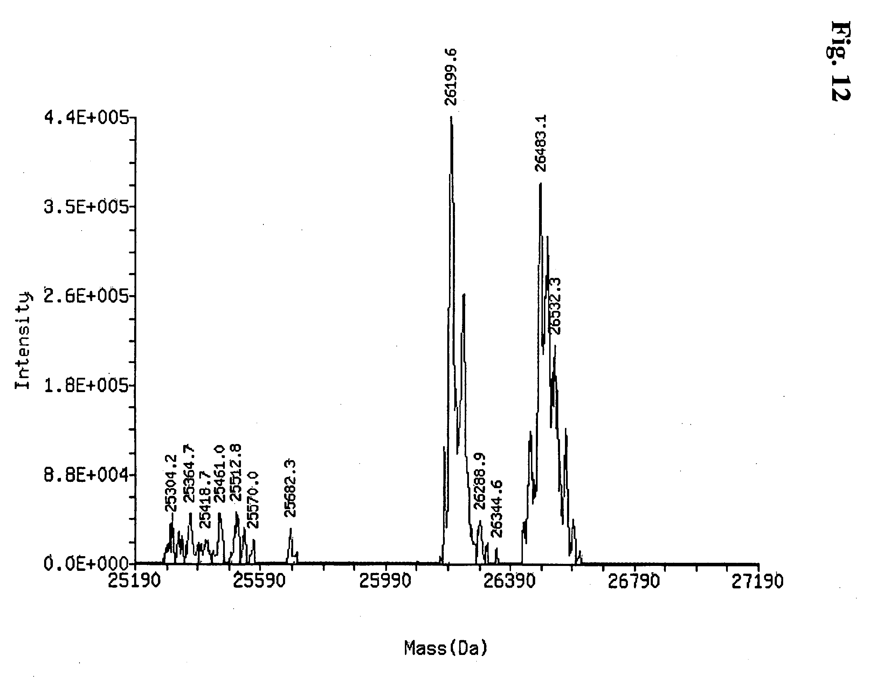

### Identification and quantification of an unknown bacterial G(+) DNA contamination in a reagent mixture (figures 8 - 12):

For this experiment the same conditions as described in example 1 were used and the unknown reagent mixture was a fluorescein labeled hybridization probe (c = 50 µM in 10 mM Tris pH = 8.0). 400 pmol of the probe was used for a PCR amplification according to Example 1. As an additional control experiment, 10 geq of *S*. *aureus* genomic DNA were spiked to this reagent mixture for a second PCR amplification in order to quantify the potential bacterial contamination.

Following the PCR amplification (see Figure 8 and 9), the content of capillary reaction vessels containing a PCR product were opened and subjected to MS analysis as described in example 1.

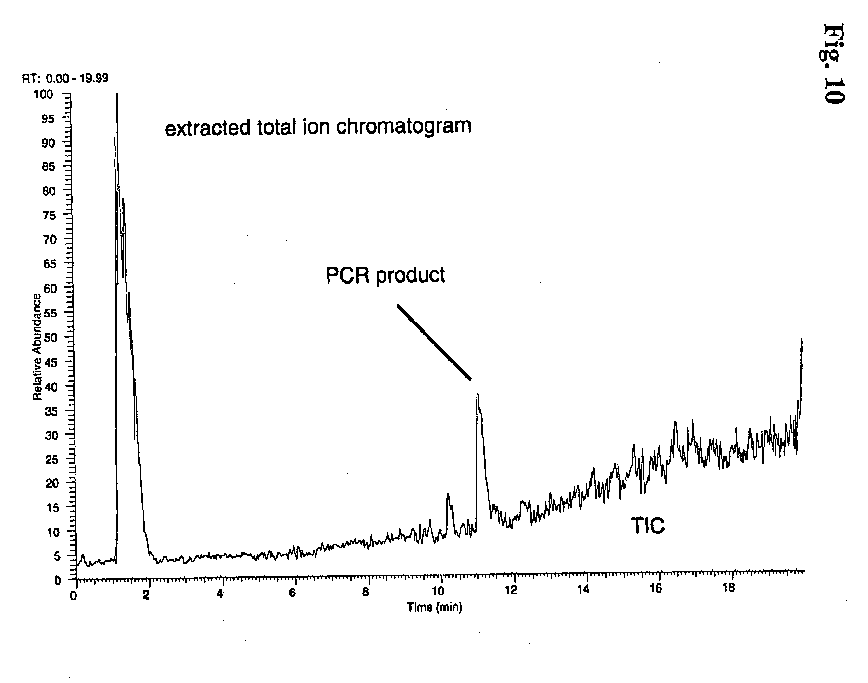

As can be seen in the PCR amplification curves (Figure 8 and 9), the reagent mixture contained a bacterial G(+) DNA contamination in the order of 10 geq. The subsequent MS analysis clearly demonstrated the presence of an amplicon of the correct size (85 bp), but the determined molecular weight did not correspond to a known *S*. *aureus* species (see Fig. 10 to 12 in comparison with Figure 2).

To summarize, in this experiment a contamination within a reaction mixture could clearly be detected and quantified that is corresponding to an unknown species of *S. aureus.* Therefore, using a classical approach with hybridization probes this unknown contamination may have remained undetected or misinterpreted.

As a control experiment to verify the conclusion above, the contamination of the reagent mixture was additionally identified by classical Sanger sequencing of the PCR product. The deviation of the molecular weight measured by the MS analysis described before and molecular weight calculated from the sequence information obtained by the classical Sanger sequencing was < 0.03 %.

## Claims

1. A method to analyze a sample with respect to the presence of biological DNA comprising the steps
a) performing an universal real-time PCR comprising the double-stranded DNA binding moiety SybrGreen, wherein potentially present biological DNA is amplified and
b) if biological DNA is amplified in step a), performing an additional analyzing step to analyze said amplified biological DNA, wherein said analyzing step is performed using a mass spectrometer (MS).

2. The method according to claim 1, wherein said sample is a biological sample, preferably blood, urine, tissue or food.

3. The method according to claim 1, wherein said sample are reagents, preferably reagents of a diagnostic kit or buffer solutions.

4. The method according to claim 1, wherein said sample are cosmetics, therapeutics or environmental samples.

5. The method according to claims 1 - 4, wherein an additional isolation step is performed prior to step a), said isolation step isolates biological DNA from said sample.

6. The method according to claims 1 - 5, wherein said universal real-time PCR in step a) is performed with at least one primer amplifying biological DNA potentially present in said sample.

7. The method according to claims 1 - 6, wherein said mass spectrometer (MS) is an electrospray ionization MS, a desorption electrospray ionization MS, a matrix-assisted laser desorption ionization MS, a fast atom bombardment MS, an ion-trap MS, triple quadrupol MS, time of flight (TOF) MS, quadrupol TOF MS, Fourier transform MS or combinations thereof.

8. The method according to claims 1 - 7, wherein two or more of said universal real-time PCRs are performed in parallel and wherein each of said two or more universal real-time PCRs comprises different primers.

## Patentansprüche

1. Verfahren zur Analyse einer Probe hinsichtlich des Vorliegens biologischer DNA, das die folgenden Schritte umfasst:
a) Durchführen einer universellen Echtzeit-PCR mit der Doppelstrang-DNA bindenden Komponente SybrGreen, wobei potentiell vorhandene biologische DNA amplifiziert wird, und,
b) falls biologische DNA in Schritt a) amplifiziert wird, Durchführen eines zusätzlichen Analyseschritts zur Analyse der amplifizierten biologischen DNA, wobei der Analyseschritt unter Verwendung eines Massenspektrometers (MS) durchgeführt wird.

2. Verfahren nach Anspruch 1, wobei es sich bei der Probe um eine biologische Probe, vorzugsweise Blut, Urin, Gewebe oder Lebensmittel, handelt.

3. Verfahren nach Anspruch 1, wobei es sich bei der Probe um Reagentien, vorzugsweise Reagentien eines diagnostischen Kits oder Pufferlösungen, handelt.

4. Verfahren nach Anspruch 1, wobei es sich bei der Probe um Kosmetika, Therapeutika oder Umweltproben handelt.

5. Verfahren nach Anspruch 1 - 4, wobei vor Schritt a) ein zusätzlicher Isolierungsschritt durchgeführt wird, bei dem biologische DNA aus der Probe isoliert wird.

6. Verfahren nach Anspruch 1 - 5, wobei die universelle Echtzeit-PCR in Schritt a) mit wenigstens einem in der Probe potentiell vorhandene biologische DNA amplifizierenden Primer durchgeführt wird.

7. Verfahren nach Anspruch 1 - 6, wobei es sich bei dem Massenspektrometer (MS) um ein Elektrospray-Ionisation-MS, ein Desorption-Elektrospray-Ionisation-MS, ein Matrix-gestütztes Laser-Desorption-Ionisation-MS, ein Schneller-Atombeschuss-MS, ein Ionenfalle-MS, ein Tripel-Quadrupol-MS, ein TOF-(Time-of-Flight-)MS, ein Quadrupol-TOF-MS, ein Fourier-Transform-MS oder Kombinationen davon handelt.

8. Verfahren nach Anspruch 1 - 7, wobei zwei oder mehr universelle Echtzeit-PCR parallel durchgeführt werden und wobei die zwei oder mehr universellen Echtzeit-PCR jeweils unterschiedliche Primer umfassen.

## Revendications

1. Procédé pour analyser un échantillon par rapport à la présence d'ADN biologique, comprenant les étapes :
a) de mise en oeuvre d'une PCR universelle en temps réel comprenant la fraction SybrGreen se liant à de l'ADN double brin, au cours de laquelle de l'ADN biologique potentiellement présent est amplifié ; et
b) lorsque de l'ADN biologique est amplifié à l'étape a), de mise en oeuvre d'une étape d'analyse supplémentaire pour analyser ledit ADN biologique amplifié, ladite étape d'analyse étant mise en oeuvre en utilisant une spectrométrie de masse (MS).

2. Procédé selon la revendication 1, dans lequel ledit échantillon représente un échantillon biologique, de préférence du sang, de l'urine, un tissu ou un aliment.

3. Procédé selon la revendication 1, dans lequel ledit échantillon représente des réactifs, de préférence des réactifs d'un nécessaire de diagnostic ou de solutions tampons.

4. Procédé selon la revendication 1, dans lequel ledit échantillon représente un échantillon cosmétique, un échantillon thérapeutique ou un échantillon environnemental.

5. Procédé selon l'une quelconque des revendications 1 à 4, dans lequel on met en oeuvre une étape d'isolation supplémentaire avant l'étape a), ladite étape d'isolation permettant d'isoler de l'ADN biologique par rapport audit échantillon.

6. Procédé selon l'une quelconque des revendications 1 à 5, dans lequel ladite PCR universelle en temps réel à l'étape a) est mise en oeuvre avec au moins une amorce amplifiant l'ADN biologique potentiellement présent dans ledit échantillon.

7. Procédé selon l'une quelconque des revendications 1 à 6, dans lequel ladite spectrométrie de masse (MS) est une MS par ionisation électrospray, une MS de désorption par ionisation électrospray, une MS ionisation laser par désorption matrice, une MS par bombardement atomique rapide, une MS par piégeage d'ions, une MS triple quadripôle, une MS à temps de vol (TOF), une MS TOF quadripôle, une MS par transformée de Fourier, ou leurs combinaisons.

8. Procédé selon l'une quelconque des revendications 1 à 7, dans lequel deux desdites PCR universelles en temps réel ou plus sont mises en oeuvre en parallèle et dans lequel chacune desdites deux PCR universelles en temps réel ou plus comprend différentes amorces.
